# EUROPEAN PATENT APPLICATION

(11) **EP 2 053 132 A1**
(43) Date of publication of application: **29.04.2009**
(21) Application number: 07020660.2
(22) Date of filing: 23.10.2007
(51) Int. Cl.: C12Q 1/68

(54) **Enrichment and sequence analysis of geomic regions**

(71) Applicant: Roche Diagnostics GmbH, 68305 Mannheim (DE); F.HOFFMANN-LA ROCHE AG, 4070 Basel (CH)
(72) Inventor: Albert, Thomas, Filchburg, WI 53711 (US); Rodesch, Matthew, Stoughton, WI 53589 (US)
(74) Representative: Bösl, Raphael Konrad

(57) **Abstract**

The present invention provides novel methods for reducing the complexity of preferably a genomic sample for further analysis such as direct DNA sequencing, resequencing or SNP calling. The methods use pre-selected immobilized oligonucleotide probes to capture target nucleic acid molecules from a sample containing denatured, fragmented (genomic) nucleic acids for reducing the genetic complexity of the original population of nucleic acid molecules.

## Description

The present application relates to the field of enrichment and analysis of nucleic acid sequences by means of capturing said sequences onto a solid support. More precisely, the present invention provides a new method to capture specific genomic regions for subsequent further analysis, if the region of interest is too large to be amplified by only one or a few PCR reactions.

### BACKGROUND OF THE INVENTION

The advent of DNA microarray technology makes it possible to build an array of millions of DNA sequences in a very small area, such as the size of a microscope slide. See, e.g., US 6,375,903 and US 5,143,854, each of which is hereby incorporated by reference in its entirety. The disclosure of US 6,375,903 enables the construction of so-called maskless array synthesizer (MAS) instruments in which light is used to direct synthesis of the DNA sequences, the light direction being performed using a digital micromirror device (DMD). Using an MAS instrument, the selection of DNA sequences to be constructed in the microarray is under software control so that individually customized arrays can be built to order. In general, MAS-based DNA microarray synthesis technology allows for the parallel synthesis of over 4 million unique oligonucleotide features in a very small area of a standard microscope slide. The microarrays are generally synthesized by using light to direct which oligonucleotides are synthesized at specific locations on an array, these locations being called features.

With the availability of the entire genomes of hundreds of organisms, for which a reference sequence has generally been deposited into a public database, microarrays have been used to perform sequence analysis on DNA isolated from such organisms. DNA microarray technology has also been applied to many areas such as gene expression and discovery, mutation detection, allelic and evolutionary sequence comparison, genome mapping and more.

Many applications require searching for genetic variants and mutations across the entire human genome that underlie human diseases. In the case of complex diseases, these searches generally result in a single nucleotide polymorphism (SNP) or set of SNPs associated with disease risk. Identifying such SNPs has proved to be an arduous and frequently fruitless task because resequencing large regions of genomic DNA, usually greater than 100 kilobases (Kb) from affected individuals or tissue samples is frequently required to find a single base change or identify all sequence variants. Accordingly, the genome is typically too complex to be studied as a whole, and techniques must be used to reduce the complexity of the genome. In this context, US 6,013,440 discloses a method wherein a nucleic acid array is used to eliminate certain types of (abundant) sequences from a genomic nucleic acid sample, wherein subsequently, the nucleic acids which have not been captured by said array are further processed.

However, alternative cost-effective and rapid methods for reducing the complexity of a genomic sample in a user defined way to allow for further processing and analysis would be a desirable contribution to the art.

### BRIEF SUMMARY OF THE INVENTION

The present invention is summarized as a novel method for reducing the complexity of a large nucleic acid sample, such as a genomic sample, cDNA library or mRNA library to facilitate further processing and genetic analysis. The method particularly uses (pre-selected) immobilized nucleic acid probes to capture target nucleic acid sequences from e.g. a genomic sample by hybridizing the sample to the probes on a solid support. Then, the captured target genomic nucleic acids are preferably washed and then eluted off of the solid support. The eluted genomic sequences, in particular, are more amenable to detailed genetic analysis than a genomic sample that has not been subjected to this procedure. Accordingly, the disclosed method provides a cost-effective, flexible and efficient approach for reducing the complexity of a genomic sample. Throughout the remainder of the description, genomic samples are used for descriptive purposes, but it is understood that other large, non-genomic samples could be subjected to the same procedures.

The solid support generally has (pre-selected) support-immobilized nucleic acid probes to capture specific nucleic acid sequences ("target nucleic acids) from e.g. a genomic sample. This may be accomplished by hybridizing e.g. a genomic sample of target nucleic acid sequence(s) against a microarray having array-immobilized nucleic acid probes directed to a specific region or specific regions of the genome. After hybridization, target nucleic acid sequences present in the sample may be enriched by washing the array and eluting the hybridized genomic nucleic acids from the array. The target nucleic acid sequence(s), preferably DNA may be amplified using, for example, non-specific ligation-mediated PCR (LM-PCR), resulting in an amplified pool of PCR products of reduced complexity compared to the original (genomic) sample.

In one aspect, the invention provides a method of reducing the complexity of e.g. a genomic sample by hybridizing the sample against e.g. a microarray having array-immobilized (pre-selected) target nucleic acid probes under preferably stringent conditions sufficient to support hybridization between the array-immobilized probes and complementary regions of the genomic sample. Then the microarray is e.g. subsequently washed under conditions sufficient to remove non-specifically bound nucleic acids. The hybridized target (genomic) nucleic acid sequences are eluted from the microarray. The eluted target sequences may optionally be amplified.

Generally, the present invention concerns a method of reducing the genetic complexity of a population of nucleic acid molecules, the method comprising the steps of:
(a) either exposing fragmented, denatured nucleic acid molecules of said population to multiple, different oligonucleotide probes that are bound on a solid support under hybridizing conditions to capture nucleic acid molecules that specifically hybridize to said probes,
   or exposing fragmented, denatured nucleic acid molecules of said population to multiple, different oligonucleotide probes under hybridizing conditions followed by binding the complexes of hybridized molecules on a solid support to capture nucleic acid molecules that specifically hybridize to said probes,
   wherein in both cases said fragmented, denatured nucleic acid molecules have an average size of about 100 to about 1000 nucleotide residues, preferably about 250 to about 800 nucleotide residues and most preferably about 400 to about 600 nucleotide residues,
(b) separating unbound and non-specifically hybridized nucleic acids from the captured molecules;
(c) eluting the captured molecules from the solid support, and
(d) optionally repeating steps (a) to (c) for at least one further cycle with the eluted captured molecules.

Preferably, the multiple, different oligonucleotide probe contain a chemical group or linker which is able to bind to a solid support.

The population of nucleic acid molecules preferably contains the whole genome or at least one chromosome of an organism or at least one nucleic acid molecule with at least about 100 kb. In particular, the size(s) of the nucleic acid molecule(s) is/are at least about 200 kb, at least about 500 kb, at least about 1 Mb, at least about 2 Mb or at least about 5 Mb, especially a size between about 100 kb and about 5 Mb, between about 200 kb and about 5 Mb, between about 500 kb and about 5 Mb, between about 1 Mb and about 2 Mb or between about 2 Mb and about 5Mb.

The organism may be selected from an animal, a plant or a microorganism, in particular from human. If only limited samples of nucleic acids, e.g. of the human genome, is available, the nucleic acids may be amplified, e.g. by whole genome amplification, prior to the method of the present invention. Prior Amplification may be necessary for performing the inventive method(s) for forensic purposes, e.g. in forensic medicine.

In a further embodiment the method comprises the step of ligating adaptor molecules to one or both, preferably both ends of the nucleic acid molecules prior or after step (a).

In another embodiment the method further comprises the step of amplifying said nucleic acid molecules with at least one primer, said primer comprises a sequence which specifically hybridizes to the sequence of said adaptor molecule(s).

In particular, the population of nucleic acid molecules is a population of genomic DNA molecules. The probes may be selected from:
- a plurality of probes that defines a plurality of exons, introns or regulatory sequences from a plurality of genetic loci,
- a plurality of probes that defines the complete sequence of at least one single genetic locus, said locus having a size of at least 100 kb, preferably at least 1 Mb, or at least one of the sizes as specified above,
- a plurality of probes that defines sites known to contain SNPs, or
- a plurality of probes that defines an array, in particular a tiling array, designed to capture the complete sequence of at least one complete chromosome.

Generally, the solid support is either a nucleic acid microarray or a population of beads.

In another aspect, the amplified target nucleic acid sequences may be sequenced, hybridized to a resequencing or SNP-calling array and the sequence or genotypes may be further analyzed.

In another aspect, the invention provides an enrichment method for target nucleic acid sequences in a genomic sample, such as exons or variants, preferably SNP sites. This can be accomplished by programming genomic probes specific for a region of the genome to be synthesized on a microarray to capture complementary target nucleic acid sequences contained in a complex genomic sample.

Specifically, the present invention is directed to a method for determining nucleic acid sequence information about at least one region of nucleic acid(s), in particular genomic nucleic acid(s), e.g. the whole genome or at least one chromosome, e.g. with a size as specified above, specifically in a sample, the method comprising the steps of:
1. performing the method(s) as described above and
2. determining the nucleic acid sequence of the captured molecules, in particular by means of performing sequencing by synthesis reactions.

In a still further aspect, the present invention is directed to a method for detecting coding region variation relative to a reference genome, in particular relative to a reference genome that comprises fragmented, denatured genomic nucleic acid molecules, the method comprising the steps of:
1. performing the method(s) as described above,
2. determining the nucleic acid sequence of the captured molecules, in particular by means of performing sequencing by synthesis reactions, and
3. comparing the determined sequence to a sequence in a database, in particular to a sequence in a database of polymorphisms in the reference genome to identify variants from the reference genome.

In a still further aspect, the present invention is directed to a kit comprising a solid support and reagents for performing a method according to the present invention. Such a kit may comprise
- a double stranded adaptor molecule, and
- a solid support with multiple, different oligonucleotid probes , wherein the probes are selected from:

- a plurality of probes that define a plurality of exons, introns or regulatory sequences from a plurality of genetic loci
- a plurality of probes that define the complete sequence of at least one single genetic locus, said locus having a size of at least 100 kb, preferably at least 1 Mb, or at least one of the sizes as specified above,
- a plurality of probes that define sites known to contain SNPs, or
- a plurality of probes that define a tiling array designed to capture the complete sequence of at least one complete chromosome.
   Preferably, the kit comprises two different double stranded adaptor molecules (A and B).
   The solid support is again either a plurality of beads or a microarray. The kit may further comprise at least one or more other components selected from DNA polymerase, T4 polynucleotide kinase, T4 DNA ligase, an array hybridization solution, an array wash solution, and/or an array elution solution.
   Other objects, advantages and features of the present invention will become apparent from the following specification taken in conjunction with the accompanying drawings.

### BRIEF DESCRIPTION OF THE SEVERAL VIEWS OF THE DRAWINGS

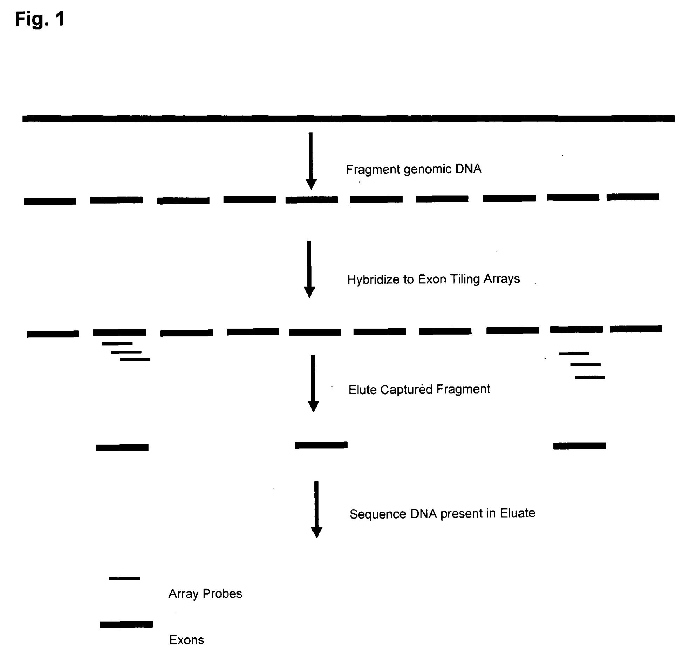

FIG. 1 is a general graphic depiction of flow diagram of a direct genomic selection process using a microarray.

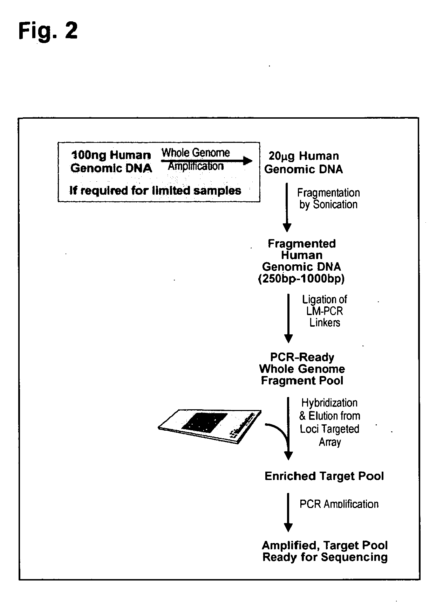

FIG. 2 is another graphic depiction of flow diagram of a direct genomic selection process using a microarray.

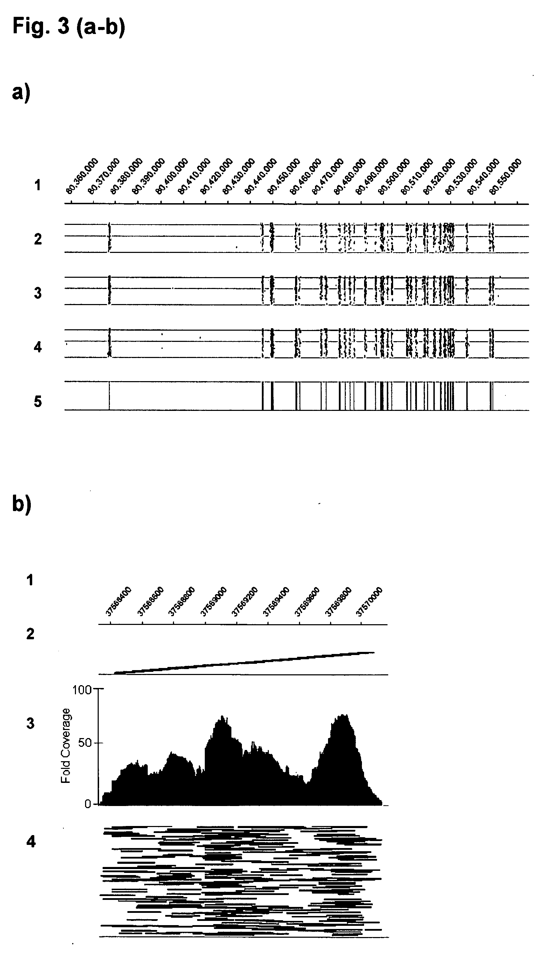

FIG 3 (a-b) show the results of a direct genomic selection process using a microarray according to Example 2. (a) Sequence read map detail of ∼190Kb of chromosome 16 from three microarray genomic selection replicates, indicating the reproducibility of targeted sequencing. Genomic DNA was from a Burkett lymphoma cell line was purified and fragmented. Tumor Sequencing Program exons (6726 genomic regions of 500bp in size), were captured using a NimbleGen oligonucleotide microarray and sequenced using a 454 sequencer. (1) Chromosome position, (2,3,4) read map of the highest BLAST score for 454 reads from three independent microarray selection and sequencing_experiments (5) regions targeted by microarray probes. (b) Sequence read map detail of ∼2,000 bases of a chromosome 17 from a microarray selection of a 2Mb contiguous region that contains the BRCA1 gene. (1) Chromosome position, (2) microarray selection probes. Probes are spaced every 10pb and staggered along the y-axis. (3) Per-base fold sequence coverage. Coverage is from 0 to 100 fold. (4) Read map of the highest blast scores for 454 sequencing reads.

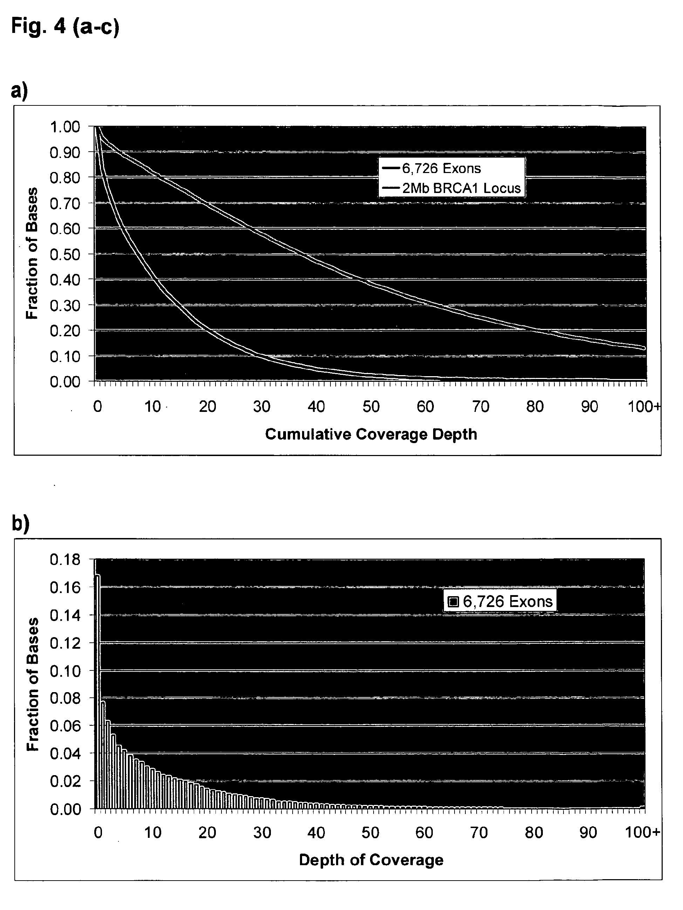

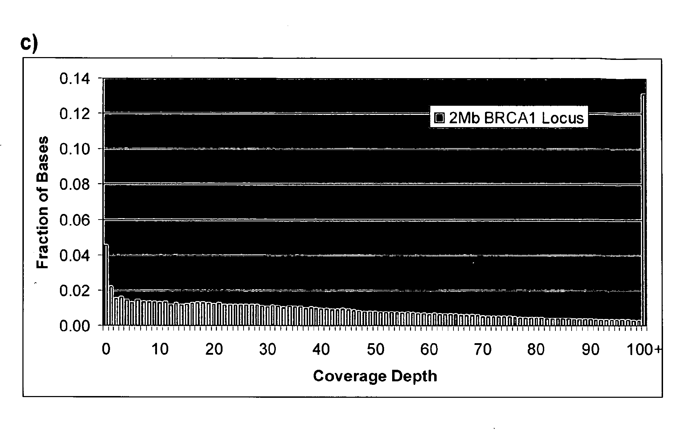

FIG. 4 (a-c) show the results of synthesizing probes on a microarray, releasing the probes from the microarray and immobilizing the probes on a support for use in a method for capturing target polynucleotides of interest. (a) Coverage depth comparison for 'Exonic' and 'Locus' selection and sequencing as disclosed in Example 2. Plot shows the fraction of bases of each aggregate target region and the corresponding cumulative depth of sequence coverage after one 454 FLX run. 'Exonic' sample represents 6,726 exon sized regions. The 2Mb BRCA1 region was targeted from positions 37,490,417 to 39,490,417 on human chromosome 17. Only the unique fraction was targeted by selection probes. (b) Histogram of per base sequence coverage depth for the Exonic exüero,emt as disclosed in Example 2. (c) Histogram of per base coverage depth for 2Mb Locus example according to Example 3.

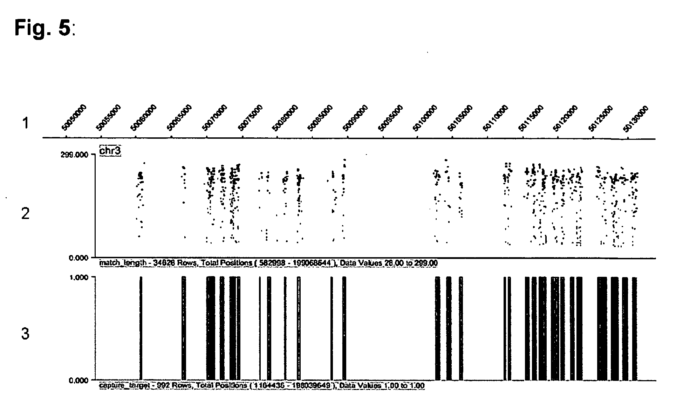

FIG. 5 illustrates a detail of the read mapping for a locus on chromosome 16 from three genomic samples. Data were generated by targeted sequencing of 6726 exons that were captured in solution. Capture oligonucleotides were cleaved and amplified from a microarray, using the protocol described in Example 4. The data presented represents an example gene map from chromosome 3. (1) chromosome position, (2) map of sequencing reads from one 454-FLX sequencing run, and (3) targeted regions. Analysis of the solution-phase capture data indicates that 83.8% of the reads map back to target regions, indicating similar performance to array-based capture protocols.

### DETAILED DESCRIPTION OF THE INVENTION

The present invention broadly relates to cost-effective, flexible and rapid methods for reducing nucleic acid sample complexity to enrich for target nucleic acids of interest and to facilitate further processing and analysis, such as sequencing, resequencing and SNP calling. The captured target nucleic acid sequences, which are of a more defined less complex genomic population are more amenable to detailed genetic analysis. Thus, the invention provides for method for enrichment of target nucleic acid in a complex nucleic acid sample.

In one embodiment, a sample containing denatured (i.e., single-stranded) nucleic acid molecules, preferably genomic nucleic acid molecules, which can be fragmented molecules, is exposed under hybridizing conditions to a plurality of oligonucleotide probes, which are immobilized on a solid support prior or after hybridization with a plurality of oligonucleotide probes to capture from the sample target nucleic acid molecules that hybridize to the immobilized probes. Non-hybridizing regions of the genome or any other sample nucleic acid remain in solution.

The nucleic acids are typically deoxyribonucleic acids or ribonucleic acids, and include products synthesized *in vitro* by converting one nucleic acid molecule type (e.g. DNA, RNA and cDNA) to another as well as synthetic molecules containing nucleotide analogues, such as PNAs. Denatured genomic DNA molecules are in particular genome-derived molecules that are shorter than naturally occurring genomic nucleic acid molecules. The skilled person can produce molecules of random- or non-random size from larger molecules by chemical, physical or enzymatic fragmentation or cleavage using well known protocols. Chemical fragmentation can employ ferrous metals (e.g., Fe-EDTA). Physical methods can include sonication, hydrodynamic force or nebulization (see European patent application EP 0 552 290). Enzymatic protocols can employ nucleases such as micrococcal nuclease (Mnase) or exo-nucleases (such as ExoI or Bal31) or restriction endonucleases. The protocol by which fragments are generated should not affect the use of the fragments in the methods. It can be advantageous during enrichment to employ fragments in a size range compatible with the post-enrichment technology in which the enriched fragments will be used. A suitable fragment size can be in the range of between about 100 and about 1000 nucleotide residues or base pairs, or between about 250 and about 800 nucleotide residues or base pairs, and can be about 400 to about 600 nucleotide residues or base pairs, in particular about 500 nucleotide residues or base pairs.

The probes correspond in sequence to at least one region of the genome and can be provided on a solid support in parallel using maskless array synthesis technology. Alternatively, probes can be obtained serially using a standard DNA synthesizer and then applied to the solid support or can be obtained from an organism and then immobilized on the solid support. After the hybridization, nucleic acids that do not hybridize, or that hybridize non-specifically to the probes are separated from the support-bound probes by washing. The remaining nucleic acids, bound specifically to the probes, are eluted from the solid support in e.g. heated water or in a nucleic acid elution buffer containing e.g. TRIS puffer and/or EDTA to yield an eluate enriched for the target nucleic acid molecules.

In some embodiments, double-stranded linkers are provided at least at one of the termini of the (genomic) nucleic acid molecules before the fragments are denatured and hybridized to the immobilized probes. In such embodiments, target nucleic acid molecules can be amplified after elution to produce a pool of amplified products having reduced complexity relative to the original sample. The target nucleic acid molecules can be amplified using for example, non-specific LM-PCR through multiple rounds of thermal cycling. Optionally, the amplified products can be further enriched by a second selection against the probes. The products of the second selection can be amplified again prior to use as described. This approach is summarized graphically in Fig. 1 and in a flow chart in Fig. 2. The linkers can be provided in an arbitrary size and with an arbitrary nucleic acid sequence according to what is required for downstream analytical applications subsequent to the complexity reduction step. The linkers can range between about 12 and about 100 base pairs, including a range between about 18 and 100 base pairs, and preferably between about 20 and 24 base pairs.

Alternatively, nucleic acid probes for target molecules can be synthesized on a solid support, released from the solid support as a pool of probes and amplified as described. The amplified pool of released probes can be covalently- or non-covalently immobilized onto a support, such as glass, metal, ceramic or polymeric beads or other solid support. The probes can be designed for convenient release from the solid support by providing, e.g., at or near the support-proximal probe termini an acid- or alkali-labile nucleic acid sequence that releases the probes under conditions of low or high pH, respectively. Various cleavable linker chemistries are known in the art. The support can be provided, e.g., in a column having fluid inlet and outlet. The art is familiar with methods for immobilizing nucleic acids onto supports, for example by incorporating a biotinylated nucleotide into the probes and coating the support with streptavidin such that the coated support non-covalently attracts and immobilizes the probes in the pool. The sample or samples are passed across the probe-containing support under hybridizing conditions such that target nucleic molecules that hybridize to the immobilized support can be eluted for subsequent analysis or other use.

In one aspect, the invention enables capturing and enriching for target nucleic acid molecules or target genomic region(s) from a complex biological sample by direct genomic selection. The invention is also useful in searching for genetic variants and mutations, such as single nucleotide polymorphisms (SNP), or set of SNPs, that underlie human diseases. It is contemplated that capture and enrichment using microarray hybridization technology is much more flexible than other methods currently available in the field of genomic enrichment, such as use of BAC (bacterial artificial chromosome) for direct genomic selection (see Lovett et al., 1991).

The invention enables targeted array-based-, shotgun-, capillary-, or other sequencing methods known to the art. In general, strategies for shotgun sequencing of randomly generated fragments are cost-effective and readily integrated into a pipeline, but the invention enhances the efficiency of the shotgun approach by presenting only fragments from one or more genomic regions of interest for sequencing. The invention provides an ability to focus the sequencing strategies on specific genomic regions, such as individual chromosomes or exons for medical sequencing purposes.

Target nucleic acid molecules can be enriched from one or more samples that include nucleic acids from any source, in purified or unpurified form. The source need not contain a complete complement of genomic nucleic acid molecules from an organism. The sample, preferably from a biological source, includes, but is not limited to pooled isolates from individual patients, tissue samples, or cell culture. As used herein, the term "target nucleic acid molecules" refers to molecules from a target genomic region to be studied. The pre-selected probes determine the range of targeted nucleic acid molecules. The skilled person in possession of this disclosure will appreciate the complete range of possible targets and associated targets.

The target region can be one or more continuous blocks of several megabases (Mb), or several smaller contiguous or discontiguous regions such as all of the exons from one or more chromosomes, or sites known to contain SNPs. For example, the solid support can support a tiling array designed to capture one or more complete chromosomes, parts of one or more chromosomes, all exons, all exons from one or more chromosomes, selected exons, introns and exons for one or more genes, gene regulatory regions, and so on. Alternatively, to increase the likelihood that desired non-unique or difficult-to-capture targets are enriched, the probes can be directed to sequences associated with (e.g., on the same fragment as, but separate from) the actual target sequence, in which case genomic fragments containing both the desired target and associated sequences will be captured and enriched. The associated sequences can be adjacent or spaced apart from the target sequences, but the skilled person will appreciate that the closer the two portions are to one another, the more likely it will be that genomic fragments will contain both portions. Still further, to further reduce the limited impact of cross-hybridization by off-target molecules, thereby enhancing the integrity of the enrichment, sequential rounds of capture using distinct but related capture probe sets directed to the target region can be performed. Related probes are probes corresponding to regions in close proximity to one another in the genome that can, therefore, hybridize to the same genomic DNA fragment.

Microarray oligonucleotides are designed to target the target region or regions of the genome. The length of individual probes is typically between 50 and 200 bases. These probes may be either designed to be overlapping probes, meaning that the starting nucleotides of adjacent probes are less than the length of a probe, or nonoverlapping probes, where the distance between adjacent probes are greater than the length of a probe. The distance between adjacent probes is generally overlapping, with spacing between the starting nucleotide of two probes varying between 1 and 100 bases. This distance can be varied to cause some genomic regions to be targeted by a larger number of probes than others. This variation can be used to modulate the capture efficiency of individual genomic regions, normalizing capture. Probes can be tested for uniqueness in the genome. To avoid non-specific binding of genomic elements to capture arrays, highly repetitive elements of the genome should be excluded from selection microarray designs using a new method that utilizes a strategy similar to the WindowMasker program developed by Morgolis (2006) to identify these regions and exclude them from probe selection. The process compared the set of probes against a pre-computed frequency histogram of all possible 15-mer probes in the human genome. For each probe, the frequencies of the 15-mers comprising the probe are then used to calculate the average 15-mer frequency of the probe. The higher the average 15-mer frequency, the more likely the probe is to lie within a repetitive region of the genome. Only probes with an average 15-mer frequency less than 100 should be used.

The nature and performance of the probes can be varied to advantageously normalize or adjust the distribution of the target molecules captured and enriched in accord with the methods. A goal of such normalization is to deliver one expressed gene per read (see Soares, et al., 1994) Normalization can be applied, for example, to populations of cDNA molecules before library construction, because the distribution of molecules in the population reflects the different expression levels of expressed genes from which the cDNA molecule populations are produced. For example, the number of sequencing reactions required to effectively analyze each target region can be reduced by normalizing the number of copies of each target sequence in the enriched population such that across the set of probes the capture performance of distinct probes are normalized, on the basis of a combination of fitness and other probe attributes. Fitness, characterized by a "capture metric," can be ascertained either informatically or empirically. In one approach, the ability of the target molecules to bind can be adjusted by providing so-called isothermal (Tm-balanced) oligonucleotide probes, as are described in U.S. Published Patent Application No. US-2005/0282209 (NimbleGen Systems, Madison, WI), that enable uniform probe performance, eliminate hybridization artifacts and/or bias and provide higher quality output. Probe lengths are adjusted (typically, about 20 to about 100 nucleotides, preferably about 40 to about 85 nucleotides, in particular about 45 to about 75 nucleotides, e.g. 45 nucleotides but optionally also more than 100 nucleotides until about 250 nucleotides) to equalize the melting temperature (e.g. Tm = 76°C, typically about 55°C to about 76°C, in particular about 72°C to about 76°C) across the entire set. Thus, probes are optimized to perform equivalently at a given stringency in the genomic regions of interest, including AT- and GC-rich regions. Relatedly, the sequence of individual probes can be adjusted, using natural bases or synthetic base analogs such as inositol, or a combination thereof to achieve a desired capture fitness of those probes. Similarly, locked nucleic acid probes, peptide nucleic acid probes or the like having structures that yield desired capture performance can be employed. The skilled artisan in possession of this disclosure will appreciate that probe length, melting temperature and sequence can be coordinately adjusted for any given probe to arrive at a desired capture performance for the probe. Conveniently, the melting temperature (Tm) of the probe can be calculated using the formula: Tm=5x(Gn+Cn)+1x(An+Tn), where n is the number of each specific base (A, T, G or C) present on the probe.

Capture performance can also be normalized by ascertaining the capture fitness of probes in the probe set, and then adjusting the quantity of individual probes on the solid support accordingly. For example, if a first probe captures twenty times as much nucleic acid as a second probe, then the capture performance of both probes can be equalized by providing twenty times as many copies of the second probe, for example by increasing by twenty-fold the number of features displaying the second probe. If the probes are prepared serially and applied to the solid support, the concentration of individual probes in the pool can be varied in the same way.

Still further, another strategy for normalizing capture of target nucleic acids is to subject the eluted target molecules to a second round of hybridization against the probes under less stringent conditions than were used for the first hybridization round. Apart from the substantial enrichment in the first hybridization that reduces complexity relative to the original genomic nucleic acid, the second hybridization can be conducted under hybridization conditions that saturate all capture probes. Presuming that substantially equal amounts of the capture probes are provided on the solid support, saturation of the probes will ensure that substantially equal amounts of each target are eluted after the second hybridization and washing.

Another normalizing strategy follows the elution and amplification of captured target molecules from the solid support. Target molecules in the eluate are denatured using, for example, a chemical or thermal denaturing process, to a single-stranded state and are re-annealed. Kinetic considerations dictate that abundant species re-anneal before less abundant species. As such, by removing the initial fraction of re-annealed species, the remaining single-stranded species will be balanced relative to the initial population in the eluate. The timing required for optimal removal of abundant species is determined empirically.

Summarizing, an embodiment of the present invention provides a new method of reducing the genetic complexity of a population of nucleic acid molecules. This method comprises
(a) either exposing fragmented, denatured nucleic acid molecules of said population to multiple, different oligonucleotide probes that are bound on a solid support under hybridizing conditions to capture nucleic acid molecules that specifically hybridize to said probes,
   or exposing fragmented, denatured nucleic acid molecules of said population to multiple, different oligonucleotide probes under hybridizing conditions followed by binding the complexes of hybridized molecules on a solid support to capture nucleic acid molecules that specifically hybridize to said probes,
   wherein (in both cases) said fragmented, denatured nucleic acid molecules have an average size of about 100 to about 1000 nucleotide residues, preferably about 250 to about 800 nucleotide residues and most preferably about 400 to about 600 nucleotide residues,
(b) separating unbound and non-specifically hybridized nucleic acids from the captured molecules;
(c) eluting the captured molecules from the solid support, preferably in an eluate pool having reduced genetic complexity relative to the original sample, and
(d) optionally repeating steps (a) to (c) for at least one further cycle with the eluted captured molecules.

In most cases, the population of nucleic molecules are molecules originated from a sample of genomic DNA (genomic nucleic acid molecules). However, it is also possible to start with a sample of cDNA or even RNA. Fragmentation can in principle be done by any method which is known in the art as already explained above. However, the fragmented denatured nucleic acid molecules should have an average size of about 100 to about 1000 nucleotide residues, preferably about 250 to about 800 nucleotide residues and most preferably about 400 to about 600 nucleotide residues. For example, this can be achieved by means of nebulization of genomic DNA (see e.g. the European patent application EP 0 552 290).

The parameters of genetic complexity reduction can be chosen almost arbitrarily, depending upon the user's desire for sequence selection, and are defined by the sequences of the multiple oligonucleotide probes. In one embodiment, said multiple probes define a plurality of exons, introns or regulatory sequences from a plurality of genetic loci. In another embodiment, said multiple probes define the complete sequence of at least one single genetic locus, said locus having a size of at least 100 kb and preferably at least 1 Mb or a size as specified above. In still another embodiment, said multiple probes define sites known to contain SNPs. In a further embodiment, said multiple probes define a tiling array. Such a tiling array in the context of the present invention is defined as being designed to capture the complete sequence of at least one complete chromosome. In this context, the term "define" is understood in such a way that the population of multiple probes comprises at least one probe for each target sequence that shall become enriched. Preferably, the population of multiple probes additionally comprises at least a second probe for each target sequence that shall become enriched, characterized in that said second probe has a sequence which is complementary to said first sequence.

The solid support according to the present invention is either a nucleic acid microarray or a population of beads. Said beads may be glass, metal, ceramic and polymeric beads. If said solid support is a microarray, it is possible to synthesize the oligonucleotide capture probes in situ directly onto said solid support. For example, the probes may be synthesized on the microarray using a maskless array synthesizer (US 6,375,903). The lengths of the multiple oligonucleotide probes may vary, are dependent on the experimental design and are limited only by the possibility to synthesize such probes. Preferably, the average length of the population of multiple probes is about 20 to about 100 nucleotides, preferably about 40 to about 85 nucleotides, in particular about 45 to about 75 nucleotides, e.g. 45 nucleotides.

If the solid support is a population of beads, the capture probes may be initially synthesized on a microarray using a maskless array synthesizer, then released or cleaved off according to known standard methods, optionally amplified and then immobilized on said population of beads according to methods known in the art. The beads may be packed into a column so that a sample is loaded and passed through the column for reducing genetic complexity. Alternatively, in order to improve the hybridization kinetics, hybridization may take place in an aqueous solution comprising the beads with the immobilized multiple oligonucleotide molecules in suspension.

In one embodiment, the multiple different oligonucleotide probes each carry a chemical group or linker, i.e. a moiety which allows for immobilization onto a solid support, also named an immobilizable group. Then the step of exposing the fragmented, denatured nucleic acid molecules of the sample to the multiple, different oligonucleotide probes under hybridizing conditions is performed in an aqueous solution and immobilization onto an appropriate solid support takes place subsequently. For example, such a moiety may be biotin which can be used for immobilization on a streptavidin coated solid support. In another embodiment, such a moiety may be a hapten like digoxygenin, which can be used for immobilization on a solid support coated with a hapten recognizing antibody, e.g. a digoxygenin binding antibody.

In a specific embodiment, the plurality of immobilized probes is characterized by normalized capture performance. The normalized capture performance is generally achieved by methods as described above, typically comprising the steps of a) ascertaining the capture fitness of probes in the probe set; and b) adjusting the quantity of at least one probe on the solid support. Alternatively, the normalized capture performance is achieved by a method comprising the steps of a) ascertaining the capture fitness of probes in the probe set; and b) adjusting at least one of the sequence, the melting temperature and the probe length of at least one probe on the solid support. Still alternatively, the normalized capture performance is achieved by a method comprising the steps of a) exposing the captured molecules to the at least one immobilized probe on the solid support under less stringent conditions than in the first exposing step such that the at least one probe is saturated, b) washing unbound and non-specifically bound nucleic acids from the solid support; and c) eluting the bound target nucleic acids from the solid support. Still alternatively, the normalized capture performance is achieved by a method comprising the steps of a) denaturing the eluted captured molecules to a single-stranded state; b) re-annealing the single-stranded molecules until a portion of the molecules are double-stranded; and discarding the double-stranded molecules and c) retaining the single-stranded molecules.

Usually at least one immobilized probe hybridizes to a genomic region of interest on nucleic acid fragments in the sample. Alternatively, the at least one immobilized probe may hybridize to sequences on target nucleic acid fragments comprising a genomic region of interest, the hybridizing sequences being separate from the genomic region of interest. Furthermore, it is also within the scope of the present invention, that at least a second hybridization step using at least one oligonucleotide probe related to but distinct from the at least one probe used in the initial hybridization is performed.

In particular, the present invention is also directed to a method for determining nucleic acid sequence information of at least one region of genomic nucleic acid in a sample, the method comprising the steps of:
- reducing the genetic complexity of a population of nucleic acid molecules according to any method as disclosed herein, and
- determining the nucleic acid sequence of the captured molecules e.g. by means of performing a sequencing reaction. Preferably, such a sequencing reaction is a sequencing by synthesis reaction.
According to this embodiment, the genomic DNA is preferably fragmented by mechanical stress. The desired average size of the DNA fragments shall be small (<= 1000 bp) and depends on the sequencing method to be applied.

Sequencing by synthesis according to the literature in the art (see e.g. Hyman, E. D., 1988) is defined as any sequencing method which monitors the generation of side products upon incorporation of a specific desoxynucleoside-triphosphate during the sequencing reaction (see e.g. Rhonaghi et al., 1998). One particular and most prominent embodiment of the sequencing by synthesis reaction is the pyrophosphate sequencing method. In this case, generation of pyrophosphate during nucleotide incorporation is monitored by means of an enzymatic cascade which finally results in the generation of a chemo-luminescent signal. For example, the 454 Genome Sequencer System (Roche Applied Science cat. No. 04 760 085 001) is based on the pyrophosphate sequencing technology. For sequencing on a 454 GS20 or 454 FLX instrument, the average genomic DNA fragment size should be in the range of 200 or 600 bp, respectively.

Alternatively, the sequencing by synthesis reaction is a terminator dye type sequencing reaction. In this case, the incorporated dNTP building blocks comprise a detectable label, which is preferably a fluorescent label that prevents further extension of the nascent DNA strand. The label is then removed and detected upon incorporation of the dNTP building block into the template/primer extension hybrid for example by means of using a DNA polymerase comprising a 3'-5' exonuclease or proofreading activity.

Advantageously, the inventive method of first reducing genomic complexity and then determining multiple sequences further comprises the step of ligating adaptor molecules to one or both, preferably both ends of the fragmented nucleic acid molecules. Adaptor molecules in the context of the present invention are preferably defined as blunt ended double stranded oligonucleotides. In addition, the inventive method may further comprise the step of amplification of said nucleic acid molecules with at least one primer, said primer comprising a sequence which corresponds to or specifically hybridizes with the sequence of said adaptor molecules.

In order to ligate adaptor molecules onto a double stranded target molecule, it is preferred that this target molecule itself is blunt ended. In order to achieve this, the double stranded target molecules are subjected to a fill-in reaction with a DNA Polymerase such as T4-DNA polymerase or Klenow polymerase in the presence of desoxynucleoside triphposphates, which results in blunt ended target molecules. In addition, e.g. T4 Polynucleotide kinase is added prior to the ligation in order to add phosphate groups to the 5' terminus for the subsequent ligation step. Subsequent ligation of the adaptors (short double stranded blunt end DNA oligonucleotides with about 3-20 base pairs) onto the polished target DNA may be performed according to any method which is known in the art, preferably by means of a T4-DNA ligase reaction.

Said ligation may be performed prior to or after the step of exposing a sample that comprises fragmented, denatured genomic nucleic acid molecules to multiple oligonucleotide probes under hybridizing conditions to capture target nucleic acid molecules that hybridize to said probes. In case ligation is performed subsequently, the enriched nucleic acids which are released from the solid support in single stranded form should be re-annealed first followed by a primer extension reaction and a fill-in reaction according to standard methods known in the art.

Ligation of said adaptor molecules allows for a step of subsequent amplification of the captured molecules. Independent from whether ligation takes place prior to or after the capturing step, there exist two alternative embodiments. In the first embodiment, one type of adaptor molecules is used. This results in population of fragments with identical terminal sequences at both ends of the fragment. As a consequence, it is sufficient to use only one primer in a potential subsequent amplification step. In an alternative embodiment, two types of adaptor molecules A and B are used. This results in a population of enriched molecules composed of three different types: (i) fragments having one adaptor (A) at one end and another adaptor (B) at the other end, (ii) fragments having adaptors A at both ends, and (iii) fragments having adaptors B at both ends.

Generation of enriched molecules according to type (i) is of outstanding advantage, if amplification and sequencing is e.g. performed with the 454 life science corporation GS20 and GSFLX instrument (see GS20 Library Prep Manual, Dec 2006, WO 2004/070007). If one of said adaptors, e.g. adaptor B carries a biotin modification, then molecules (i) and (iii) can e.g. be bound on streptavidin (SA) coated magnetic particles for further isolation and the products of (ii) washed away. In case the enriched and SA-immobilized DNA is single stranded following elution from the capture array/solid support, it is advantageous to make the DNA double-stranded. In this case primers complementary to adaptor A may be added to the washed SA pull down products. Since moieties that are B-B (iii above) do not have A or its complement available, only A-B adapted and SA captured products will be made double stranded following primer-extension from an A complement primer. Subsequently, the double stranded DNA molecules that have been bound to said magnetic particles are thermally or chemically (e.g. NaOH) denatured in such a way that the newly synthesized strand is released into solution. Due to the tight biotin/streptavidin bonding, for example, molecules with only two adaptors B will not be released into solution. The only strand available for release is the A-complement to B-complement primer-extension synthesized strand. Said solution comprising single stranded target molecules with an adaptor A at one end and an adaptor B at the other end can e.g. subsequently be bound on a further type of beads comprising a capture sequence which is sufficiently complementary to the adaptor A or B sequences for further processing.

In case of the Genome Sequencer workflow (Roche Applied Science Catalog No. 04 896 548 001), in a first step, (clonal) amplification is performed by means of emulsion PCR. Thus, it is also within the scope of the present invention, that the step of amplification is performed in the form of an emulsion PCR. The beads carrying the clonally amplified target nucleic acids may then become arbitrarily transferred into a picotiter plate according to the manufacturer's protocol and subjected to a pyrophosphate sequencing reaction for sequence determination.

Thus, the methods according to the present invention enable sequence determinations for a variety of different applications. For example, the present invention also provides a method for detecting coding region variation relative to a reference genome, preferably in a sample that comprises fragmented, denatured genomic nucleic acid molecules, the method comprising the steps of:
- performing the method(s) as described above,
- determining nucleic acid sequence of the captured molecules, and
- comparing the determined sequence to a database, in particular to a database of polymorphisms in the reference genome to identify variants from the reference genome.

In a further major aspect, the present invention also provides a kit for performing a method or part of a method according to the present invention as disclosed herein. Thus, the present invention is also directed to a kit comprising
- a (first) double stranded adaptor molecule, and
- solid support with multiple probes, wherein the multiple probes are selected from:
   - a plurality of probes that defines a plurality of exons, introns or regulatory sequences from a plurality of genetic loci
   - a plurality of probes that defines the complete sequence of at least one single genetic locus, said locus having a size of at least 100 kb, preferably at least 1 Mb or a size as specified herein,
   - a plurality of probes that defines sites known to contain SNPs, and
   - a plurality of probes that defines an array, in particular a tiling array especially designed to capture the complete sequence of at least one complete chromosome.

Preferably, the kit contains two different double stranded adaptor molecules. The solid support can be either a plurality of beads or a microarray as disclosed herein.

In one embodiment, such a kit further comprises at least one or more compounds from a group consisting of DNA polymerase, T4 polynucleotide kinase, T4 DNA ligase, an array hybridization solution, e.g. as disclosed herein, an array wash solution, in particular a wash solution with SSC, DTT and optionally SDS, e.g. Wash Buffer I (0.2x SSC, 0.2% (v/v) SDS, 0.1mM DTT), Wash Buffer II (0.2x SSC, 0.1mM DTT) and/or Wash Buffer III (0.05x SSC, 0.1mM DTT), and/or an array elution solution, e. g water or a solution containing TRIS buffer and/or EDTA.

In a further specific embodiment, not mutually exclusive to the embodiment disclosed herein, the kit comprises a second adaptor molecule. At least one oligonucleotide strand of said first or second adaptor molecule may carry a modification, which allows for immobilization onto a solid support. For example, such a modification may be a Biotin label which can be used for immobilization on a streptavidin coated solid support. Alternatively, such a modification may be a hapten like digoxygenin, which can be used for immobilization on a solid support coated with a hapten recognizing antibody.

As used herein, the term "hybridization" is used in reference to the pairing of complementary nucleic acids. Hybridization and the strength of hybridization (i.e., the strength of the association between the nucleic acids) is affected by such factors as the degree of complementary between the nucleic acids, stringency of the conditions involved, the Tₘ of the formed hybrid, and the G:C ratio of the nucleic acids. While the invention is not limited to a particular set of hybridization conditions, stringent hybridization conditions are preferably employed. Stringent hybridization conditions are sequence-dependent and will differ with varying environmental parameters (e.g., salt concentrations, and presence of organics). Generally, "stringent" conditions are selected to be about 5°C to 20°C lower than the thermal melting point (Tm) for the specific nucleic acid sequence at a defined ionic strength and pH. Preferably, stringent conditions are about 5°C to 10°C lower than the thermal melting point for a specific nucleic acid bound to a complementary nucleic acid. The Tm is the temperature (under defined ionic strength and pH) at which 50% of a nucleic acid (e.g., tag nucleic acid) hybridizes to a perfectly matched probe.

Similarly, "stringent" wash conditions are ordinarily determined empirically for hybridization of each set of tags to a corresponding probe array. The arrays are first hybridized (typically under stringent hybridization conditions) and then washed with buffers containing successively lower concentrations of salts, or higher concentrations of detergents, or at increasing temperatures until the signal-to-noise ratio for specific to non-specific hybridization is high enough to facilitate detection of specific hybridization. Stringent temperature conditions will usually include temperatures in excess of about 30°C, more usually in excess of about 37°C, and occasionally in excess of about 45°C. Stringent salt conditions will ordinarily be less than about 1000 mM, usually less than about 500 mM, more usually less than about 150 mM. For further information see e.g., Wetmur et al. (1966) and Wetmur (1991).

"Stringent conditions" or "high stringency conditions," as defined herein, can be hybridization in 50% formamide, 5x SSC (0.75 M NaCl, 0.075 M sodium citrate), 50 mM sodium phosphate (pH 6.8), 0.1 % sodium pyrophosphate, 5x Denhardt's solution, sonicated salmon sperm DNA (50 mg/ml), 0.1 % SDS, and 10% dextran sulfate at 42° C, with washes at 42°C in 0.2x SSC (sodium chloride/sodium citrate) and 50% formamide at 55°C, followed by a wash with 0.1x SSC containing EDTA at 55°C.

By way of example, but not limitation, it is contemplated that buffers containing 35% formamide, 5x SSC, and 0.1% (w/v) sodium dodecyl sulfate are suitable for hybridizing under moderately non-stringent conditions at 45°C for 16-72 hours. Furthermore, it is envisioned that the formamide concentration may be suitably adjusted between a range of 20-45% depending on the probe length and the level of stringency desired. Also encompassed within the scope of the invention is that probe optimization can be obtained for longer probes (>>50mer), by increasing the hybridization temperature or the formamide concentration to compensate for a change in the probe length. Additional examples of hybridization conditions are provided in several sources, including: "Direct selection of cDNAs with large genomic DNA clones," in Molecular Cloning: A Laboratory Manual (2001).

The following examples are provided as further non-limiting illustrations of particular embodiments of the invention.

### EXAMPLES

### Example 1 - Discovery of New Polymorphisms and Mutations in Large Genomic Regions

This generic example describes how to perform selection that allows for rapid and efficient discovery of new polymorphisms and mutations in large genomic regions. Microarrays having immobilized probes are used in one- or multiple rounds of hybridization selection with a target of total genomic DNA, and the selected sequences are amplified by LM-PCR (see Figs. 1 and 2).

### a) Preparation of the genomic DNA and double-stranded linkers

DNA is fragmented using sonication to an average size of ∼500 base pairs.

A reaction to polish the ends of the sonicated DNA fragments is set up:

| | |
|---|---|
| DNA fragments | 41 µl |
| T4 DNA Polymerase | 20 µl |
| T4 DNA polymerase reaction mix | 20 µl |
| Water | 10 µl |

The reaction is incubated at 11°C for 30 min. The reaction is then subjected to phenol/chloroform extraction procedures and the DNA is recovered by ethanol precipitation. The precipitated pellet is dissolved in 10 µl water (to give a final concentration of 2 µg/µl).

Two complementatry oligonucleotides are annealed to create a double-stranded linker, by mixing the following:

| | |
|---|---|
| Oligonucleotide 1 (1µg/µl) | 22.5 µl |
| **(5'-CTCGAGAATTCTGGATCCTC-3')** | |
| Oligonucleotide 2 (1µg/µl) | 22.5 µl |
| **(5'-GAGGATCCAGAATTCTCGAGTT-3')** | |
| 10x annealing buffer | 5 µl |
| Water | to 50 µl |

The reaction is heated at 65°C for 10 min; then allowed to cool at 15-25°C for 2 h. The length of the 2 complementary oligonucleotides 1 and 2 is between 12 and 24 nucleotides, and the sequence is selected depending upon the functionality desired by the user. The double-stranded linker is then purified by column chromatography through a Sephadex G-50 spin column. The purified linker solution is then concentrated by lyophilization to a concentration of 2 µg/µl.

### b) Ligation of linkers to genomic DNA fragments

The following reaction to ligate the linkers to genomic DNA fragments is set up. The reaction is incubated at 14°C overnight.

| | |
|---|---|
| Annealed linkers from Step a) (20 µg) | 10 µl |
| Genomic DNA from Step a) (10 µl) | 5 µl |
| T4 DNA ligase | 10 U |
| 10x ligation buffer | 2 µl |
| Water | to 20 µl |

The reaction volume is adjusted to 500 µl with water and the ligated genomic DNA is purified using a QIAquick PCR purification kit. The purified DNA is stored at a concentration of 1 µg/µl.

### c) Primary selection and capture of hybrids

To prepare the genomic DNA sample for hybridization to the microarray, linkered genomic DNA (10µg) is resuspended in 3.5µl of nuclease-free water and combined with 31.5µl NimbleGen Hybridization Buffer (NimbleGen Systems Inc., Madison, WI), 9µl Hybridization Additive (NimbleGen Systems), in a final volume of 45µl. The samples are heat-denatured at 95°C for 5 minutes and transferred to a 42°C heat block.

To capture the target genomic DNA on the microarray, samples are hybridized to NimbleGen CGH arrays, manufactured as described in US6,375,903. Maskless fabrication of capture oligonucleotides on the microarrays is performed by light-directed oligonucleotide syntheses using a digital micromirror as described in Singh-Gasson et al. (1999). Gene expression analysis using oligonucleotide arrays produced by maskless photolithography is described in Nuwaysir et al. (2002). All references are herein incorporated in their entirety. Hybridization is performed in a MAUI Hybridization System (BioMicro Systems, Inc., Salt Lake City, UT) according to manufacturer instructions for 16 hours at 42°C using mix mode B. Following hybridization, arrays are washed twice with Wash Buffer I (0.2x SSC, 0.2% (v/v) SDS, 0.1mM DTT, NimbleGen Systems) for a total of 2.5 minutes. Arrays are then washed for 1 minute in Wash Buffer II (0.2x SSC, 0.1mM DTT, NimbleGen Systems) followed by a 15 second wash in Wash Buffer III (0.05x SSC, 0.1mM DTT, NimbleGen Systems).

To elute the genomic DNA hybridized to the microarray, the arrays are incubated twice for 5 minutes in 95°C water. The eluted DNA is dried down using vacuum centrifugation.

### d) Amplification of the primary selected DNA

The primary selected genomic DNA is amplified as described below. Ten separate replicate amplification reactions are set up in 200µl PCR tubes. Only one oligonucleotide primer is required because each fragment has the same linker ligated to each end:

| Reagents | |
|---|---|
| Template: primary selection | |
| material | 5 µl |
| Oligonucleotide 1 (200 ng/µl) | 1 µl |
| **(5'-CTCGAGAATTCTGGATCCTC-3')** | |
| dNTPs (25mM each) | 0.4 µl |
| 10x PfuUltra HF DNA polymerase | |
| Reaction buffer | 5 µl |
| PfuUltra HF DNA polymerase | 2.5 U |
| Water | to 50 µl |

The reactions are amplified according to the following program:

| Cycle number | Denaturation | Annealing | Polymerization |
|---|---|---|---|
| 1 | 2 min at 95°C | | |
| 2-31 | 30 s at 95°C | 30 s at 55°C | 1 min at 72°C |

The reaction products are analyzed by agarose gel electrophoresis. The amplification products are purified using a QIAquick PCR purification kit. The eluted samples are pooled and the concentration of amplified primary selected DNA is determined by spectrophotometry. A volume of DNA in the pool equivalent to 1 µg is reduced to 5 µl in a speed vacuum concentrator. 1 µl (at least 200 ng) of the primary selected material is set aside for comparison with the secondary selection products. As necessary, subsequent rounds of enrichment are performed by further rounds of array hybridization and amplification of the eluted sample.

### e) Preparation of target oligonucleotide probes for release from microarray and immobilization on support

Probes are synthesized on a microarray, then are released using a base-labile Fmoc (9-fluorenylmethyloxycarbonyl) group. The probes are labeled with biotin and are then immobilized onto the surface of a streptavidin solid support using known methods for covalent or non-covalent attachment.

Optionally, prior to immobilization onto the solid support, the synthesized probes are amplified using LM-PCR, Phi29 or other amplification strategy to increase the amount of the synthesized probes by virtue of inserting sequences upon them that facilitate their amplification. This material can now be used for direct sequencing, array based resequencing, genotyping, or any other genetic analysis targeting the enriched region of the genome by employing solution phase hybridization and SA mediated capture of the hybridization products.

### Example 2 - Array-Targeted Resequencing

A series of high-density oligonucleotide microarrays that capture short segments that correspond to 6,726 individual gene exon regions of at least 500 base pairs were chosen from 660 genes distributed about the human genome (sequence build HG17) (∼ 5Mb of total sequence) were synthesized according to standard NimbleGen microarray manufacturing protocols (see references in Example 1). Overlapping microarray probes of more than 60 bases each on the array spanned each target genome region, with a probe positioned each 10 bases for the forward strand of the genome.

Highly-repetitive genomic regions were excluded by design from the capture microarrays, to reduce the likelihood of non-specific binding between the microarrays and genomic nucleic acid molecules. The strategy for identifying and excluding highly-repetitive genomic regions was similar to that of the WindowMasker program (Morgulis, A. et al. (2006), incorporated by reference herein as if set forth in its entirety). The average 15-mer frequency of each probe was calculated by comparing the frequencies of all 15-mers present in the probe against a pre-computed frequency histogram of all possible 15-mer probes in the human genome. The likelihood that the probe represents a repetitive region of the genome increases as the average 15-mer frequency increases. Only probes having an average 15-mer frequency below 100 were included on the capture microarrays.

To test the reproducibility of the capture system, the 'exonic' design was first used to capture fragmented genomic DNA from a human cell line (Burkitt's Lymphoma, NA04671 (Coriell)) using the method shown schematically in Fig. 2. The genomic DNA (20 µg) was subjected to whole genome amplification (WGA; using Qiagen service (Hilden, Germany)). 20 µg of the whole genome amplification product was then treated with Klenow fragment of DNA polymerase I (NEB, Beverly MA) to generate blunt-ends. The blunt-ended fragments were sonicated to generate fragments of about 500 base pairs and then 5' phosphorylated with polynucleotide kinase (NEB). Oligonucleotide linkers
(5'-Pi-GAGGATCCAGAATTCTCGAGTT-3' and 5'-CTCGAGAATTCTGGATCCTC-3') were annealed and ligated to the ends of the 5' phosphorylated fragments:

The linker-terminated fragments were denatured to produce single stranded products that were exposed to the capture microarrays under hybridization conditions in the presence of 1x hybridization buffer (NimbleGen Systems, Inc., Madison WI) for approximately 65 hours at 42°C with active mixing using a MAUI hybridization station (NimbleGen Systems, Inc.). Single-stranded molecules that did not hybridize were washed from the microarrays under stringent washing conditions, 3 x 5 minutes with Stringent Wash Buffer (NimbleGen) and then rinsed with Wash Buffers 1, 2, and 3 (NimbleGen). Fragments captured on the microarrays were immediately eluted with 2 x 250 µl of water at 95°C, dried and resuspended for amplification by LM-PCR using a primer complementary to the linker ligated earlier.

To quantify enrichment of the exonic regions, eight random regions were selected for quantitative PCR (qPCR). These regions were amplified using the following primers:

| | |
|---|---|
| Region 1 | F: 5'-CTACCACGGCCCTTTCATAAAG-3' |
| | R: 5'-AGGGAGCATTCCAGGAGAGAA-3' |
| | |
| Region 2 | F: 5'-GGCCAGGGCTGTGTACAGTT-3' |
| | R: 5'-CCGTATAGAAGAGAAGACTCAATGGA-3' |
| | |
| Region 3 | F: 5'-TGCCCCACGGTAACAGATG-3' |
| | R: 5'-CCACGCTGGTGATGAAGATG-3' |
| | |
| Region 4 | F: 5'-TGCAGGGCCTGGGTTCT-3' |
| | R: 5'-GCGGAGGGAGAGCTCCTT-3' |
| | |
| Region 5 | F: 5'-GTCTCTTTCTCTCTCTTGTCCAGTTTT-3' |
| | R: 5'-CACTGTCTTCTCCCGGACATG-3' |
| | |
| Region 6 | F: 5'-AGCCAGAAGATGGAGGAAGCT-3' |
| | R: 5'-TTAAAGCGCTTGGCTTGGA-3' |
| | |
| Region 7 | F: 5'-TCTTTTGAGAAGGTATAGGTGTGGAA-3' |
| | R: 5'-CAGGCCCAGGCCACACT-3' |
| | |
| region 8 | F: 5'-CGAGGCCTGCACAGTATGC-3' |
| | R: 5'-GCGGGCTCAGCTTCTTAGTG-3' |

After a single round of microarray capture, the enriched, amplified samples and control genomic DNA, that was fragmented, linker-ligated and LM-PCR amplified, but not hybridized to a capture array, were compared using an ABI 7300 real time PCR system (Applied Biosystems, Foster City, CA) measuring SYBR green fluorescence according to manufacturer's protocols. An average of 378-fold enrichment was achieved for three replicate exonic capture products. The theoretical maximum enrichment level was 600 fold (3,000 Mb in the genome and 5 Mb of total sequence).

Samples eluted from the capture microarrays were ligated to 454-sequencing-compatible linkers, amplified using emulsion PCR on beads and sequenced using the 454 FLX sequencing instrument (454, Branford CT). Because each sequenced fragment also contained the 20bp LM-PCR linker used immediately after microarray elution, the majority of 454 sequencing reads contained that linker sequence. DNA sequencing of the three replicates on the 454 FLX instrument generated 63 Mb, 115 Mb, and 93 Mb of total sequence. Following *in-silico* removal of the linker sequence, each sequencing read was compared to the entire appropriate version of the Human Genome using BLAST analysis (Altschul, S.F. et al. (1990), incorporated herein by reference as if set forth in its entirety) using a cutoff score of e = 10⁻⁴⁸, tuned to maximize the number of unique hits. Reads that did not uniquely map back to the genome (between 10 and 20%) were discarded. The rest were considered "captured sequences". Captured sequences that, according to the original BLAST comparison, map uniquely back to regions within the target regions were considered "sequencing hits". These were then used to calculate the % of reads that hit target regions, and the fold sequencing coverage for the entire target region. Data was visualized using SignalMap software (NimbleGen).

BLAST analysis showed that 91%, 89%, and 91% of reads, respectively, mapped back uniquely to the genome; 75%, 65%, and 77% were from targeted regions and 96%, 93%, and 95% of target sequences contained at least one sequence read (Table 1, upper three rows). This represents an average enrichment of about 400 fold. Fig. 4a illustrates a detail of the read mapping for chromosome 16 from the three genomic samples. Line 1 depicts the chromosomal position, lines 2-4 shows the read maps of the samples, and line 5 highlights the regions targeted by the microarray probes. Fig. 4 shows the cumulative per-base coverage (Fig. 4a) and coverage histograms (Fig. 4b) for replicate 3. The median per-base coverage for each sample was 5-, 7- and 7-fold coverage respectively.

**Table 1:**

| DNA Sample | qPCR Fold Enrichment | FLX - Yield (Mb) | Percentage of Reads Mapped Uniquely to the Genome | Percentage of Total Reads That Mapped to Selection Targets | Median Fold Coverage for Target Regions |
|---|---|---|---|---|---|
| NA04671 | 318 | 63.1 | 91% | 75% | 5 |
| NA04671 | 399 | 115 | 89% | 65% | 7 |
| NA04671 | 418 | 93.0 | 91% | 76% | 7 |
| HapMap CEPH | 217 | 77.6 | 88% | 74% | 7 |
| HapMap JPT | 153 | 96.7 | 84% | 66% | 8 |
| HapMap CHB | 240 | 52.8 | 83% | 59% | 4 |
| HapMap YRI | 363 | 81.3 | 53% | 38% | 4 |

### Example 3 - Sequence Variation Captured by Genomic Enrichment and Resequencing

To ascertain the ability to discern variation in the human genome, genomic DNA samples from four cell types in the human HapMap collection (CEPH/ NA11839, CHB/NA18573, JPT/NA18942, YRI/NA18861, Coriell) were captured on the exon arrays of the prior examples, eluted and sequenced, as disclosed herein, except that the genomic DNAs were not whole genome amplified before capture. The capture results (shown in Table 1, rows 4-7) were similar to those above, except that sequence coverage was consistently more uniform than before, suggesting a bias introduced during WGA.

The sequence from the four HapMap samples was assembled and mutations were identified and compared to the HapMap SNP data for each sample (Tables 1 and 2). The total number of positions in the target regions that were genotyped in the HapMap project was 8103 (CEU), 8134 (CHB), 8134 (JPT), 8071 (YRI) for each of the four genomes. Of these, most (∼6000) sites were homozygous for the reference genome allele. The number of known variant alleles (homozygous or heterozygous) is listed in the second row of Table 2. These positions were analyzed for coverage and to determine whether the allele(s) were found in the captured DNA.

**Table 2:**

| Pop/Indiv | CEPH/ NA11839 | CHB/NA18573 | JPT/ NA18942 | CEPH/ NA11839 |
|---|---|---|---|---|
| # Known variant alleles | 2235 | 2257 | 2206 | 2334 |
| Stringency of at least one read per known variant HapMap allele | | | | |
| Positions with ≥ 1 read | 2176 (97.3%) | 2104 (93.2%) | 2168 (98.2%) | 2133 (91.3%) |
| Variant alleles found in ≥ 1 read | 2071 (92.6%) | 1922 (85.1%) | 2080 (94.2%) | 1848 (79.1%) |
| False negative rate | 7.4% | 14.9% | 5.8% | 20.9% |
| Stringency of at least two reads per known variant HapMap allele | | | | |
| Positions with ≥ 1 read | 2176 (97.3%) | 2104 (93.2%) | 2168 (98.2%) | 2133 (91.3%) |
| Variant alleles found in ≥ 2 reads | 1907 (85.3%) | 1569 (69.5%) | 1939 (87.8%) | 1469 (62.9%) |
| False negative rate | 14.7% | 30.5% | 12.2% | 37.1% |

Between 94% and 79% of known variant positions among the HapMap samples were identified with at least one sequence read, which was expected, based upon the overall sequence coverage. There was no apparent biasing against alleles not present on the capture array when coverage of targets that contained 0, 1 or >1 known variants, (7.95, 8.48, and 8.82 fold coverage respectively) were compared.

There is considerable interest in the analysis of large contiguous genomic regions. Capture microarray series that target single long segments from 200kb - 5Mb surrounding the human BRCA1 gene were tested with the NA04671 DNA. For array series used to capture the BRCA1 gene locus, five genomic regions of increasing size (200 kb, 500kb, 1Mb, 2Mb, and 5Mb) surrounding the BRCA1 gene locus were chosen from the human genome sequence (build HG18). Attributes of the locus-capture arrays are shown in Table 3. The average probe tiling density is the average distance between the start of one probe and the start of the next probe.

**Table 3:**

| BRCA1 Region Size | Average Selection Probe Tiling Density (base pairs) | Chromosome 17 coordinates (HG18) |
|---|---|---|
| 200kb | 1bp | 38,390,417 - 38,590,417 |
| 500kb | 1bp | 38,240,417 - 38,740,417 |
| 1Mb | 2bp | 37,990,417 - 38,990,417 |
| 2Mb | 3bp | 37,490,417 - 39,490,417 |
| 5Mb | 7bp | 35,990,417 - 40,990,417 |

Table 4 shows that all capture targets performed well, with up to 140 Mb of raw sequence generated in a single sequencing machine run, generating ∼18 fold coverage, from a 5 Mb capture region. Fig. 4b provides sequence read map details for the locus-specific capture and sequencing. Line 1 depicts the chromosome position of 2000 bases on human chromosome 17, line 2 shows the location of the probes, spaced every 10 base pairs and staggered along the Y axis, the chart at 3 shows the per-base fold sequence coverage, which ranges between 0 and 100 percent, and item 4 depicts the read map of the highest BLAST scores for 454 sequencing reads. Fig. 5 displays cumulative per-base sequence coverage (Fig. 5a) and a sequence coverage histogram (Fig. 4c) for the BRCA1 2Mb region. The percentage of reads that map to the target sequence increased with the size of the target region.

**Table 4:**

| Tiling Size (kb) | Average Selection Probe Tiling Density | FLX -Yield (Mb) | Percentage of Reads Mapped Uniquely to the Genome | Percentage of Total Reads That Mapped to Selection Targets | Median fold coverage of Unique Portion of Region |
|---|---|---|---|---|---|
| 200 | 1bp | 102 | 55% | 14% | 79 |
| 500 | 1bp | 85.0 | 61% | 36% | 93 |
| 1,000 | 2bp | 96.7 | 56% | 35% | 38 |
| 2000 | 3bp | 112.6 | 81% | 60% | 37 |
| 5,000 | 7bp | 140 | 81% | 64% | 18 |

These data illustrate the power of microarray-based direct selection methods for enriching targeted sequences. The inventor used a programmable high-density array platform with 385,000 probes that were readily able to capture at least 5Mb of total sequence. In addition to the specificity of the assay, the high yields of the downstream DNA sequencing steps are consistently superior to the routine average performance using non-captured DNA sources. This is attributed to the capture-enrichment process providing a useful purification of unique sequences away from repeats and other impurities that can confound, for example, the first emulsion PCR step of the 454 sequencing process.

### Example 4 - Solution Phase Capture and Resequencing

The sample of Examples 2 and 3 was tested using capture probes synthesized upon, then liberated from, a solid support such that the enrichment was advantageously executed in solution phase. Standard microarray designs (e.g. the BRCA1 200K Tiling array and human exon capture arrays of the prior examples) were modified by adding terminal 15mer primer sequences containing an MlyI recognition site, which facilitates enzymatic primer removal while leaving the capture oligonucleotide sequence intact.

Arrays were synthesized by adding chemical phosphorylating reagent (Glen Research) after the initial T₅ linker and before the 3' primer sequence. Three individual couplings were performed to maximize subsequent cleavage of capture probes from the arrays.

The array-immobilized capture probes were treated with 30% ammonium hydroxide (NH₄OH, Aldrich). After synthesis, arrays were placed in a humid chamber and ∼700 µl of NH₄OH was applied to the synthesis area at ambient room temperature for 20 minutes to cleave the probes from the array. The NH₄OH remained largely within the confines of the synthesis area because of hydrophobicity differences between the reaction area and the surrounding glass. The solution was removed using a pipette and was retained. An additional 700 µl of fresh NH₄OH was applied to the surface. The process was repeated for a total of 3x (60 min and 2.1 ml total). Cleaved oligonucleotide capture probes were then dried by centrifugation under vacuum under standard conditions known in the art

The cleaved capture probes were amplified under standard conditions. Dried probes were resuspended in 30 µl deionized water (DIH₂O) and aliquoted into 30 individual PCR runs as follows:

| | | |
|---|---|---|
| 10x buffer | 2.5 µl | 95°C for 15min |
| 25mM dNTPs | 0.125 µl | 95°C for 20s |
| 40µM Primer 1a | 1.25 µl | 48°C for 45s |
| 40µM Primer 1b (biotinylated) | 1.25 µl | 72°C for 20s |
| | | repeat 30x |
| HotStart Taq | 0.25 µl | 4°C forever |
| MgCl | 1 µl | |
| Sample | 1 µl | |
| H₂O | 17.625 µl | |
| Total volume | 25 µl | |

Primer 1a:
5'- /Biotin/AGT CAG AGT CGC CAC - 3'
Primer 1b:
5'- TGC CGG AGT CAG CGT - 3'

PCR reactions were cleaned using the QiaQuick Nucleotide Removal Kit (Qiagen), dried down, and resuspended in 20 µl DIH₂O. Typical yield after cleanup is ∼400-700ng/rxn by Nanodrop. Amplicons may be checked on a 3% agarose gel. Depending on quantity requirements of capture probes, additional 'standard' PCR rounds were optionally performed as above with ∼200ng of sample per reaction. Amplicons were purified and characterized as above.

The final round of amplification of the capture probes was performed using asymmetric PCR. The protocol was as above, except that while the biotinylated primer concentration remained the same, the non-biotinylated primer concentration was reduced to 0.001x of the original concentration. The protocol was extended to 35 cycles to allow for non-exponential amplification. Amplicons were dried, resuspended in 20µl DIH₂O, and characterized.

The genomic DNA sample was prepared per standard protocol; 20 µg of WGA linkered sample was dried with 100 µg Cot-1 DNA and resuspended in 7.5 µl hybridization buffer and 3µl formamide. A 2 µg aliquot of capture probes was dried and resuspended in 4.5 µl DIH₂O. The sample solution was mixed with the capture probe solution and incubated at 95°C for 10 minutes. The mixture was then transferred to a PCR tube and placed in a thermal cycler for 3 days at 42°C for hybridization to form duplexes.

After hybridization, the duplexes were bound to paramagnetic beads (Dynal). 25 µl of beads were washed three times in 2x BW buffer (10mM TrisHCl, 1mM EDTA, 2M NaCl), and the beads were resuspended in the hybridization mixture. Binding occurred over 45 minutes at 42°C with occasional gentle mixing.

Bound beads were isolated using a magnet and washed briefly with 40 µl Wash Buffer I, incubated for 2 x 5 minutes in 47°C stringent wash buffer, washed with Wash Buffer I for ∼2 minutes at ambient room temperature, with Wash Buffer II for ∼1 minute, and with Wash Buffer III for -30 seconds.

To elute the captured fragments, the solution containing beads in Wash Buffer III was transferred to a 1.5 ml Eppendorf tube. The beads were isolated with a magnet. The wash buffer was removed and ∼100ul of 95°C DIH₂O is added. The solution was incubated at 95°C for 5 minutes, after which the beads were bound with a magnet and gently washed with 95°C DIH₂O. The wash liquid was then removed and retained, and replaced with fresh 95°C DIH₂O. Incubation and washing was repeated for a total of 3 times (15minutes, ∼300 µl eluate). After the final wash, the Eppendorf tube containing eluate is placed on a magnetic stand for ∼5 minutes to isolate any beads aspirated during elution. The solution was dried at high heat in a fresh Eppendorf tube. The eluted captured fragments were resuspended in 263µl DIH₂O prior to standard LM-PCR.

Following LM-PCR, the captured fragments were subjected to standard ultra-deep sequencing using the 454 FLX platform, as above. Alternatively, LM-PCR can be avoided by ligating 454 sequencing adapter sequences to the pre-enrichment sample. In that case, the eluted enriched sequences can be piped directly into the emulsion PCR for ultra-deep sequencing.

Fig. 5 illustrates a detail of the read mapping for chromosome 16 from a genomic sample captured by solution hybridization. Line 1 depicts the chromosome position, line 2 shows sequencing reads from one 454-FLX sequencing run and line 3 shows the targeted regions. The data indicated that 83.8% of the reads map back to target regions, which is comparable and indistinguishable from results obtained using array-based capture protocols.

It is understood that certain adaptations of the invention described in this disclosure are a matter of routine optimization for those skilled in the art, and can be implemented without departing from the spirit of the invention, or the scope of the appended claims.

### List of References

Altschul, S.F. et al. (1990) J. Mol. Biol. 215,403-410
Hyman, E. D. (1988), Anal. Biochem. 174, 423-436
Lovett et al. (1991) PNAS USA, 88, 9628-9632
Morgulis, A. et al. (2006) Bioinformatics, 15, 134-41
Nuwaysir, E.F., et al., (2002) Genome Res. 12, 1749-1755
Rhonaghi et al. (1998), Science 281, 363-365
Soares, et al. (1994) PNAS, 91, 9228-9232
Singh-Gasson, S., et al. (1999) Nat. Biotechnol. 17, 974-978
Wetmur (1991) Critical Reviews in Biochemistry and Molecular Biology, 26(34):227-59
Wetmur et al. (1966) J. Mol. Biol., 31, 349-70
"Direct selection of cDNAs with large genomic DNA clones," in Molecular Cloning: A Laboratory Manual (eds. Sambrook, J. & Russell, D.W.) Chapter 11 Protocol 4, pages 11.98-11.106 (Cold Spring Harbor Laboratory Press, Cold Spring Harbor, New York, USA, 2001)
EP 0 552 290
US 2005/0282209
US 5,143,854
US 6,013,440
US 6,375,903
WO 2004/070007

## Claims

1. A method of reducing the genetic complexity of a population of nucleic acid molecules, the method comprising the steps of:
(a) either exposing fragmented, denatured nucleic acid molecules of said population to multiple, different oligonucleotide probes that are bound on a solid support under hybridizing conditions to capture nucleic acid molecules that specifically hybridize to said probes,
or exposing fragmented, denatured nucleic acid molecules of said population to multiple, different oligonucleotide probes under hybridizing conditions followed by binding the complexes of hybridized molecules on a solid support to capture nucleic acid molecules that specifically hybridize to said probes,
wherein said fragmented, denatured nucleic acid molecules have an average size of about 100 to about 1000 nucleotide residues, preferably about 250 to about 800 nucleotide residues and most preferably about 400 to about 600 nucleotide residues,
(b) separating unbound and non-specifically hybridized nucleic acids from the captured molecules;
(c) eluting the captured molecules from the solid support, and
(d) optionally repeating steps (a) to (c) for at least one further cycle with the eluted captured molecules.

2. The method according to claim 1, wherein the multiple, different oligonucleotide probes each contain a chemical group or linker being able to bind to a solid support.

3. The method according to at least one of the claims 1-2, wherein said population of nucleic acid molecules contains the whole genome or at least one chromosome of an organism or at least one nucleic acid molecule with a size of at least about 200 kb, at least about 500 kb, at least about 1 Mb, at least about 2 Mb or at least about 5 Mb, especially a size between about 100 kb and about 5 Mb, between about 200 kb and about 5 Mb, between about 500 kb and about 5 Mb, between about 1 Mb and about 2 Mb or between about 2 Mb and about 5Mb.

4. The method according to at least on of the claims 1-3 further comprising the step of ligating adaptor molecules to one or both, preferably both ends of the nucleic acid molecules prior or after the step (a).

5. The method according to claim 4 further comprising the step of amplification of said nucleic acid molecules with at least one primer, said primer comprising a sequence which specifically hybridizes to the sequence of said adaptor molecule(s).

6. The method according to at least one of the claims 1-5, wherein said population of nucleic acid molecules is a population of genomic DNA molecules.

7. The method according to claim 6, wherein said probes are selected from:
- a plurality of probes that defines a plurality of exons, introns or regulatory sequences from a plurality of genetic loci,
- a plurality of probes that defines the complete sequence of at least one single genetic locus, said locus having a size of at least 100 kb, preferably at least 1 Mb, or at least one of the sizes as specified in claim 3,
- a plurality of probes that defines sites known to contain single nucleotide polymorphisms (SNPs), or
- a plurality of probes that defines an array, in particular a tiling array, designed to capture the complete sequence of at least one complete chromosome.

8. The method according to at least one of the claims 1-7 wherein said solid support is either a nucleic acid microarray or a population of beads.

9. A method for determining nucleic acid sequence information about at least one region of nucleic acid(s), in particular genomic nucleic acid(s), the method comprising the steps of:
1. reducing the genetic complexity of a population of nucleic acid molecules according to a method of at least one of the claims 1-8, and
2. determining the nucleic acid sequence of the captured molecules, in particular by means of performing sequencing by synthesis reactions.

10. A method for detecting coding region variation relative to a reference genome, the method comprising the steps of:
1. reducing the genetic complexity of a population of nucleic acid molecules according to a method of at least one of the claims 1-8,
2. determining the nucleic acid sequence of the captured molecules, and
3. comparing the determined sequence to sequences in a database of the reference genome, in particular to sequences in a database of polymorphisms in the reference genome to identify variants from the reference genome.

11. A kit comprising
- double stranded adaptor molecules, and
- a solid support with multiple, different oligonucleotid probes, wherein said probes are selected from:
- a plurality of probes that define a plurality of exons, introns or regulatory sequences from a plurality of genetic loci,
- a plurality of probes that define the complete sequence of at least one single genetic locus, said locus having a size of at least 100 kb, preferably at least 1 Mb, or at least one of the sizes as specified in claim 3,
- a plurality of probes that define sites known to contain SNPs, or
- a plurality of probes that define a tiling array designed to capture the complete sequence of at least one complete chromosome.

12. The kit according to claim 11, wherein the kit contains two different double stranded adaptor molecules.

13. The kit according to claim 11 or 12, wherein said solid support is either a plurality of beads or a microarray.

14. The kit according to at least on of the claims 11-13, further comprising at least one or more components selected from DNA polymerase, T4 polynucleotide kinase, T4 DNA ligase, an array hybridization solution, an array wash solution, and/or an array elution solution.


SEQUENCE LISTING

<110>  Roche Diagnostics GmbH / Hoffmann-La Roche AG

<120>  ENRICHMENT AND SEQUENCE ANALYSIS OF GENOMIC REGIONS

<130>  R65003EP

<160>  23

<170>  PatentIn version 3.3

<210>  1
<211>  22
<212>  DNA
<213>  Artificial

<220>
<223>  Primer qPCR

<400>  1
ctaccacggc cctttcataa ag                                              22

<210>  2
<211>  21
<212>  DNA
<213>  Artificial

<220>
<223>  Primer qPCR

<400>  2
agggagcatt ccaggagaga a                                               21

<210>  3
<211>  20
<212>  DNA
<213>  Artificial

<220>
<223>  Primer qPCR

<400>  3
ggccagggct gtgtacagtt                                                 20

<210>  4
<211>  26
<212>  DNA
<213>  Artificial

<220>
<223>  Primer qPCR

<400>  4
ccgtatagaa gagaagactc aatgga                                          26

<210>  5
<211>  19
<212>  DNA
<213>  Artificial

<220>
<223>  Primer qPCR

<400>  5
tgccccacgg taacagatg                                                    19


<210>  6
<211>  20
<212>  DNA
<213>  Artificial

<220>
<223>  Primer qPCR

<400>  6
ccacgctggt gatgaagatg                                                   20


<210>  7
<211>  17
<212>  DNA
<213>  Artificial

<220>
<223>  Primer qPCR

<400>  7
tgcagggcct gggttct                                                      17


<210>  8
<211>  18
<212>  DNA
<213>  Artificial

<220>
<223>  Primer qPCR

<400>  8
gcggagggag agctcctt                                                     18


<210>  9
<211>  27
<212>  DNA
<213>  Artificial

<220>
<223>  Primer qPCR

<400>  9
gtctctttct ctctcttgtc cagtttt                                           27


<210>  10
<211>  21
<212>  DNA
<213>  Artificial

<220>
<223>  Primer qPCR

<400>  10
cactgtcttc tcccggacat g                                                 21


<210>  11
<211>  21
<212>  DNA
<213>  Artificial

<220>
<223>  Primer qPCR

<400>  11
agccagaaga tggaggaagc t                                                21


<210>  12
<211>  19
<212>  DNA
<213>  Artificial

<220>
<223>  Primer qPCR

<400>  12
ttaaagcgct tggcttgga                                                   19


<210>  13
<211>  26
<212>  DNA
<213>  Artificial

<220>
<223>  Primer qPCR

<400>  13
tcttttgaga aggtataggt gtggaa                                           26


<210>  14
<211>  17
<212>  DNA
<213>  Artificial

<220>
<223>  Primer qPCR

<400>  14
caggcccagg ccacact                                                     17


<210>  15
<211>  19
<212>  DNA
<213>  Artificial

<220>
<223>  Primer qPCR

<400>  15
cgaggcctgc acagtatgc                                                   19


<210>  16
<211>  20
<212>  DNA
<213>  Artificial

<220>
<223>  Primer qPCR

<400>  16
gcgggctcag cttcttagtg                                                  20

<210>  17
<211>  20
<212>  DNA
<213>  Artificial

<220>
<223>  DNA-Oligonukleotid

<400>  17
ctcgagaatt ctggatcctc                                                    20


<210>  18
<211>  22
<212>  DNA
<213>  Artificial

<220>
<223>  DNA-Oligonukleotid

<400>  18
gaggatccag aattctcgag tt                                                 22


<210>  19
<211>  20
<212>  DNA
<213>  Artificial

<220>
<223>  DNA Oligonukleotid

<400>  19
ctcgagaatt ctggatcctc                                                    20


<210>  20
<211>  22
<212>  DNA
<213>  Artificial

<220>
<223>  DNA-Oligonukleotid

<400>  20
gaggatccag aattctcgag tt                                                 22


<210>  21
<211>  20
<212>  DNA
<213>  Artificial

<220>
<223>  DNA-Oligonukleotid

<400>  21
ctcgagaatt ctggatcctc                                                    20


<210>  22
<211>  15
<212>  DNA
<213>  Artificial

<220>
<223>  Biotin DNA-Oligonukleotid

<400>  22
agtcagagtc gccac                                                        15


<210>  23
<211>  15
<212>  DNA
<213>  Artificial

<220>
<223>  DNA-Oligonukleotid

<400>  23
tgccggagtc agcgt                                                        15